# EUROPEAN PATENT SPECIFICATION

(11) **EP 2 213 386 B1**
(45) Date of publication and mention of the grant of the patent: **07.06.2017**
(21) Application number: 08842143.3
(22) Date of filing: 21.10.2008
(51) Int. Cl.: B09C 1/10, A62D 3/02, B09C 1/02, B09C 1/08, C12N 1/20, A62D 101/45, C12R 1/06

(54) **METHOD FOR WASHING SOIL CONTAINING CYANOGEN COMPOUND**
VERFAHREN ZUM WASCHEN VON EINE CYANVERBINDUNG ENTHALTENDEM ERDREICH
PROCÉDÉ POUR LAVER UN SOL CONTENANT UN COMPOSÉ DE CYANOGÈNE

(30) Priority: 22.10.2007 JP 2007273699
(43) Date of publication of application: 04.08.2010
(73) Proprietor: Showa Denko K.K., Tokyo 105-8518 (JP)
(72) Inventor: FUJITA, Ichiro, Kawasaki-shi Kanagawa 210-0858 (JP); YONEDA, Tadashi, Chiba-shi Chiba 267-0056 (JP)
(74) Representative: Strehl Schübel-Hopf & Partner
(86) International application number: PCT/JP2008/069019
(87) International publication number: WO 2009/054368

(56) References cited:
- JP-A- 2006 255 572
- US-A- 5 302 287
- US-A- 5 302 287
- US-B2- 6 620 611
- ANDHALE M S ED - HOUGHTON ET AL: "Microbial Degradation of Cyanide Containing Effluent from a Dye Industry", 30 December 1987 (1987-12-30), BIODETERIORATION 7, LONDON [U.A.] : ELSEVIER, GB, PAGE(S) 213 - 218, XP008134452, ISBN: 978-1-85166-221-0 * abstract * * page 213, line 36 - page 218, line 5 *
- F.J.Alguacil and Y.Merino: "Bioremediation of inorganic contaminants", Rev.Metal.Madrid, vol. 34, no. 5 31 December 1998 (1998-12-31), pages 428-436, XP002715317, Retrieved from the Internet: URL:http://revistademetalurgia.revistas.cs ic.es/index.php/revistademetalurgia/articl e/view/810/821 [retrieved on 2013-10-23]
- ANDHALE M.S.: 'Biodeterior', 1988 article 'Microbial Degradation of Cyanide Containing Effluent from a Dye Industry', pages 213 - 218, XP008134452
- MEEUSSEN J.C.L. ET AL.: 'Dissolution Behavior of Iron Cyanide (Prussian Blue) in Contaminated Soils' ENVIRON. SCI. TECHNOL. vol. 26, no. 9, 1992, pages 1832 - 1838, XP000296136

## Description

The present invention relates to a method for remediation of soil containing a cyanogen compound using microorganism. Specifically, the present invention relates to a method for remediation of soil containing a cyanogen compound using a microorganism and a biodegradable chelating agent promoting the elution of a cyanogen compound which is difficult to extract from soil (hereinafter referred to as a "difficult-to-extract" cyanogen compound).

### TECHNICAL FIELD

The enforcement of Soil Contamination Countermeasures Act in 2003 triggered an increase in the number of the cases of remediation of the soil polluted with a cyanogen compound. With respect to a method for remediation of the polluted soil, bio-remediation using the degrading activity of a microorganism has been studied.

Since a cyanogen compound has the property of forming a difficult-to-extract complex in the presence of metal ions, when a cyanogen compound is contained in the soil, it forms a difficult-to-extract complex due to the metal such as iron which is naturally present in the soil.

In the bio-remediation, it is difficult to efficiently decompose the difficult-to-extract complex with the form as is, and therefore not only the degrading capability of the microorganism but development of technology for solubilizing the difficult-to-extract cyanogen complex is also required to establish an efficient and reliable remediation method.

With respect to the degradation and removal of the difficult-to-extract cyanogen complex in countermeasures against soil contamination with a cyanogen compound, the microorganism belonging to the Fusarium genus having capability of degrading the cyanogen complex has been proposed (see Patent publication No. JP 3685583; Patent Document 1). However, Patent Document 1 only shows degradability of the cyanogen complex in a liquid phase using a mixed solvent and mentions nothing about the remediation performance of the microorganism in the soil.

As a method for efficiently decontaminating the cyanogen compound contained in the soil by bio-remediation, a method for controlling the pH of the contaminated soil under an weakly acid condition using a mineral acid and an organic acid and supplying the nutrient source of sugars except the nitrogen source (see JP-A-2007-75670; Patent Publication 2). However, Patent Document 2 mentions nothing about the efficiency of degrading the difficult-to-extract cyanogen complex.

In addition, as a method taking in a viewpoint of remediation by solubilizing the cyanogen complex, a method of adding water containing dissolved oxygen, NOx-N (nitrite-nitrogen and nitrate-nitrogen) and the like to the polluted soil containing a cyanogen compound and the bivalent iron ions; oxidizing the bivalent iron ions to trivalent iron ions to thereby convert them into soluble complex ions; and degrading the complex using the microorganisms in the soil is known (see JP-A-2006-255572; Patent Document 3). However, as to the remediation performance, Patent Document 3 only describes treatment of the soil in which the total cyanogen concentration is as low as 2 mg/kg (2 ppm), and the remediation capability is not known against the soil having a total cyanogen concentration of several tens of ppm which can be subject to the practical bio-remediation.

For the above reason, there has been demand for establishment of a method which enables efficient and reliable reduction of the cyanogen compound contained in the soil including the difficult-to-extract portions.

Patent Document 1: Japanese Patent No. 3685583
Patent Document 2: Laid-open Japanese patent publication No. 2007-75670
Patent Document 3: Laid-open Japanese patent publication No. 2006-255572
US 6 620 611 B2 discloses a method for remediation of soil containing a cyanogen compound comprising adding a biodegradable chelating agent to the soil and degrading the cyanogen compound using microorganisms, according to the preamble of claim 1.

### DISCLOSURE OF THE INVENTION

### Problems to be Solved by the Invention

Accordingly, an objective of the present invention is to provide a method for remediation of the soil containing a cyanogen compound which enables efficient and reliable reduction of the cyanogen compound contained in the soil including the difficult-to-extract portions.

### Means to Solve Problems

The present inventors intensively studied to solve the above-mentioned problems. As a result, they have found that the cyanogen compound contained in the soil can be effectively and reliably cleansed by using the microorganism which belongs to the genus Arthrobacter which is capable of degrading the cyanogen compound and serves as a degradation-accelerating factor, and accomplished the present invention.

That is, the present invention relates to a method for remediation of the soil containing a cyanogen compound described in [1] to [3] below.
[1] A method for remediation of a soil containing a difficult-to-extract cyanogen compound comprising adding a biodegradable chelating agent to the soil to thereby make the cyanogen compound elute and degrading the eluted cyanogen compound using the microorganism Arthrobacter sp. No. 5 strain having the accession number FERM BP-11019.
[2] The method for remediation of a soil as described in [1] above, wherein the biodegradable chelating agent to be added is one or more of the members among citric acid, gluconic acid, tartaric acid, oxalic acid, succinic acid, carboxymethyl tartronic acid, carboxymethyloxy succinic acid, aspartate diacetic acid, L-glutamic acid diacetic acid and salts thereof.
[3] The method for remediation of a soil as described in [2] above, wherein the biodegradable chelating agent to be added is at least one selected from the group consisting of citric acid and citrate.

### EFFECTS OF THE INVENTION

The method for remediation of the soil containing a cyanogen compound of the present invention enables remediation of the cyanogen compound contained in the soil efficiently and reliably.

### BEST MODE FOR CARRYING OUT THE INVENTION

The present invention is described in more details hereinafter.

### [Bacterium having high degrading activity of a cyanogen compound]

The microorganism used in the method for remediation of the soil of the present invention is Arthrobacter sp. No. 5 strain separated from the soil by using a medium where ferricyanide potassium is the only carbon source and energy source.

The observed morphology and physiological properties of Arthrobacter sp. No. 5 strain are described below:
Morphology: polymorphic bacillus;
Gram's stain: positive;
Spores: asporogenic;
Motility: nonmotile;
Requirement for oxygen: aerobic;
Oxydase: negative;
Catalase: positive;
OF (Oxidation Fermentation) test: the strain did not grow in the test medium;
Colony color: no specific colony pigment was produced.

Arthrobacter sp. No. 5 strain was identified by analyzing the base sequence of the DNA in 16SrRNA region amplified through Polymerase Chain Reaction (PCR) method using ABI PRISM 310 Genetic Analyzer (Applied Bisosystems), comparing the obtained sequence with those registered in the International Nucleotide Sequence Databases (DDBJ/EMBL/GenBank) and in the database of MicroSeq Analysis Software (Applied Biosystems) and furthermore by drawing a family tree among related species by Neighborhood Joining (NJ) method using MicroSeq Analysis Software.

As a result, Arthrobacter sp. No. 5 strain was found to be closest to Arthrobacter oxydans and Arthrobacter polychromogenes showing 0.88% of base sequence diversity among the species.

**[Table 1]**

| Bacterium name | Sequence diversity |
|---|---|
| Arthrobacter oxydans | 0.88 % |
| Arthrobacter polychromogenes | 0.88 % |
| Arthrobacter sulfonivorans | 1.09 % |

The present strain was named Arthrobacter sp. No. 5 strain and deposited in National Institute of Advanced Industrial Science and Technology, International Patent Organism Depositary (Center No. 6, 1-1, Higashi 1-chome, Tsukuba-shi, Ibaraki 305-8566 JAPAN) under Accession No. FERM P-21400) on October 18, 2007 and then internationally deposited in National Institute of Advanced Industrial Science and Technology, International Patent Organism Depositary under Accession No. FERM BP-11019 on September 16, 2008.

### [Cultivation of microorganism]

There is no particular limitation on the cultivation of the microorganism used in the present invention as long as it is a well-known method.

As an example of the carbon source of the medium to culture the microorganism, sugars such as glucose, sucrose, fructose and blackstrap molasses can be used singly or in combination thereof at a concentration of from 0.1 w/v% to 30 w/v% generally, preferably from about 1 w/v% to about 10 w/v%.

As an example of the nitrogen source of the culture medium, peptone, meat extract, yeast extract, ammonia, ammonium sulfate, ammonium chloride, ammonium phosphate, ammonium nitrate, sodium nitrate, potassium nitrate and urea can be used singly or in combination thereof at a concentration of from 0.1 w/v% to 30 w/v% generally, preferably from about 1 w/v% to about 10 w/v%.

In addition, phosphates such as potassium hydrogen phosphate and potassium dihydrogen phosphate; metal salt such as magnesium sulfate, ferrous sulfate, calcium acetate and manganese chloride; vitamins; amino acids; and nucleic acids, biotin such as thiamin and the like can be added to improve the growth of the bacterium.

The cultivation can be carried out under aerated stirred culture or under aerobic conditions at a temperature of from 20°C to 40°C, preferably from 25°C to 35°C. Preferable pH for the culture is in the range from 5 to 10, preferably from 7 to 8, which can be easily adjusted by adding acid or alkali.

The tank used in the cultivation step is not particularly limited as long as it is a fermentation tank capable of mixing and dispersing the bacterium during culture with culture medium components under aeration.

### [Soil containing a cyanogen compound and cyanogen determination]

The soil as an object of remediation in the present invention contains a cyanogen compound: i.e. an inorganic cyanogen compound such as iron cyanide complex, copper cyanide complex, nickel cyanide complex, potassium cyanide and sodium cyanide and an organic cyanogen compound having a nitrile group.

When a cyano complex is contained in the soil, it forms a difficult-to-extract precipitate due to the metal such as iron which is naturally present in the soil, resulting in that soluble and difficult-to-extract cyanogen compounds coexist in the soil.

In order to decontaminate cyanogen compounds including difficult-to-extract portions in the present invention, cyanogen determination is conducted not only on the eluting concentration of total cyanide eluted from the soil (i.e. total eluting CN; according to "Measurement method regarding soil eluting test" in Announcement No. 18, 2003 of Ministry of Environment; unit: mg/L; the eluting standard provided by Soil Contamination Countermeasures Act <0.1) and free cyanide content in the soil (i.e. contained free CN; according to "Measurement method regarding soil eluting survey" in Announcement No. 19, 2003 of Ministry of Environment; unit: mg/L; the contained free cyanide standard provided by Soil Contamination Countermeasures Act ≤50) in the official method provided by Soil Contamination Countermeasures Act in Japan, but also on the concentration of total cyanide content in the soil as a Bottom Sediment Survey method (i.e. total contained CN; according to Bottom Sediment Survey of KANSUIKAN No. 127 bureau notification in 1988; unit: mg/kg). Furthermore, the total difficult-to-extract cyanide concentration in the soil (i.e. difficult-to-extract CN; unit: mg/kg) was also determined by the calculation formula of total contained CN - total eluting CN x 9 to use it in evaluation. The calculation formula for the total concentration of difficult-to-extract cyanide is to be determined as the mass per the dry soil (mg/kg). The total eluting CN indicates the concentration in the filtrate of the 10% soil suspension in water. Therefore, the CN in the 90% water is assumed to be contained in the 10% soil suspended in the water, the total eluting CN is multiplied by 9 in the calculation formula to calculate the value per soil for unit conversion.

**[Table 2]**

| Names in the analysis of the present study | Unit | Method of analysis | standard provided by Soil Contamination Countermeasures |
|---|---|---|---|
| Total eluting CN | mg/l | Soil Contamination Countermeasures Act; Soil eluting analysis method | below detection limit (< 0.1) |
| Contained free CN | mg/kg (dry soil) | Soil Contamination Countermeasures Act; Soil eluting analysis method | ≤ 50 |
| Total contained CN | mg/kg (dry soil) | Bottom Sediment Survey method (total contained CN in the soil) | None |
| Total difficult-to-extract CN | mg/kg (dry soil) | Calculated value = total contained CN - total eluting CN x 9 | None |

### [Soil remediation as a goal of the present invention]

The goal level of the soil remediation of the present invention is the level which enables removal of the risk that the total eluting CN is detected due to the elution from the total contained CN: i.e. total contained CN < 1 mg/kg in numerical terms. The level is based on the fact that the measuring object is the filtrate of the elute at a concentration of 10% soil in water, in which the detection limit is 0.1 mg/l, and therefore it can be concluded that at a condition of "the total contained CN < 1 mg/kg, the total eluting CN falls below the detection limit.

The cyanogen concentration contained in the soil as an object of the remediation in the present invention is not particularly limited, and the remediation can be conducted at the total contained CN of 1 mg/kg to 100 mg/kg, which is a concentration in a general bioremediation.

### [Soil treatment method]

The soil treatment method of the present invention is conducted by adding the microorganism, nutrient source, biodegradable chelating agent and the like to the soil. The addition method to the soil and the implementation scale are not particularly limited and the soil treatment can be carried out by the known method in the field of soil remediation.

The microorganism to be added to the soil may be in the form of the culture solution or diluent with water.

Though the nutrient source and biodegradable chelating agent to be added may be in either form of an aqueous solution or powder, it is desirable to be added in the form of an aqueous solution in consideration of the penetration and diffusion of the components into the soil.

When the nutrient source or biodegradable chelating agent is added in liquid form, it is allowed to leave the soil as it is or to extract the increased water from the soil.

The treatment method is carried out under ambient temperature conditions. However, in the case where the remediation performance is quantitatively measured on a laboratory scale, the treatment can be conducted by placing a soil sample in a constant-temperature bath and the like and thereby controlling the temperature at from 5°C to 50°C, preferably from 15°C to 35°C.

### [Decomposition of a cyanogen compound in the soil]

Decomposition of a cyanogen compound in the soil can be carried out by adding to the soil the microorganisms having the cyanide degradation bacterium. The microorganism to be used is Arthrobacter sp. No. 5 strain.

There are no particular limitations on the concentration of the microorganism to be added to the soil as long as the concentration allows the expression of the cyanide degradation activity. However, in consideration of taking balance between the diffusion/penetration into the soil and the degradation activity, the concentration is preferably at 10⁶ cells/g to 10⁹ cells/g, more preferably at 10⁷ cells/g to 10⁸ cells/g.

A nutrient source may be added so as to maintain the cell numbers and the degradation activity of the added microorganism. The nutrient source to be added is not particularly limited as long as it can maintain the cell numbers and the degradation activity of the microorganism and, as an example of the carbon source of the medium to culture the microorganism, sugar such as glucose, sucrose, fructose and blackstrap molasses can be used singly or in combination thereof at a concentration of from 0.001 w/w% to 2.0 w/w% generally, preferably from about 0.01 w/w% to about 0.05 w/w%.

As an example of the nitrogen source, peptone, meat extract, yeast extract, ammonia, ammonium sulfate, ammonium chloride, ammonium phosphate, ammonium nitrate, sodium nitrate, potassium nitrate and urea can be used singly or in combination thereof at a concentration of from 0.001 w/w% to 2.0 w/w% generally, preferably from about 0.01 w/w% to about 0.05 w/w%.

In addition, phosphates such as potassium hydrogen phosphate and potassium dihydrogen phosphate; metal salt such as magnesium sulfate, ferrous sulfate, calcium acetate and manganese chloride; vitamins; amino acids; and nucleic acids, biotin such as thiamin and the like can be added as needed.

The timing of adding a nutrient source is not particularly limited and a nutrient source may be added separately before adding the microorganism, added concurrently with the microorganism, or added separately after adding the microorganism. The number of times the nutrient source is added is not limited either.

### [Solubilization of difficult-to-extract cyanogen compound]

Solubilization of a difficult-to-extract cyanogen compound can be carried out by adding a biodegradable chelating agent. The chelating agent to be used is not particularly limited as long as it is biodegradable. As an example of the chelating agent, citric acid, gluconic acid, tartaric acid, oxalic acid, succinic acid, carboxymethyl tartronic acid, carboxymethyloxy succinic acid, aspartate diacetic acid, L-glutamic acid diacetic acid and salts thereof can be used singly or in combination thereof at a concentration in soil of from 0.001 w/w% to 2.0 w/w% generally, preferably from about 0.01 w/w% to about 0.05 w/w%. The particularly preferable components of the chelating agent are citric acid and salts thereof. The timing of adding a biodegradable chelating agent is not particularly limited and the chelating agent may be added separately before adding the microorganism, added concurrently with the microorganism, or added separately after adding the microorganism. The number of times the chelating agent is added is not limited either. When the cyanide degradation bacteria exists in the soil, the chelating agent may be used without being combined with the addition of the bacteria. However, the most preferable condition is using Arthrobacter sp. No. 5 strain and potassium citrate in combination.

The chelating agent to be used is not particularly limited and may be a free-acid type agent or a metal-salt type agent. In order to keep the pH in the soil neutral, it is also possible to employ a method of using the free-acid type and the metal-salt type chelating agents in combination.

The reason why to restrict the chelating agent to a biodegradable one is to prevent the residue accumulation in the soil and to minimize the environmental impact.

In the soil remediation method of the present invention, the mechanism to realize solubilization of the difficult-to-extract cyanogen compound is attributed to the result that the iron ion, which is a cause for insolubilization of the iron ferrocyanide, for example, which was altered from the soluble potassium ferricyanide due to the bivalent iron ion in the soil, is taken up into the chelating agent.

### EXAMPLES

Hereinafter, the present invention will be explained in more detail with reference to Examples and Comparative Examples.

### Example 1: Culture of Arthrobacter sp. No. 5 strain

After cultivating Arthrobacter sp. No. 5 strain (Accession No. in National Institute of Advanced Industrial Science and Technology, International Patent Organism Depositary: FERM BP-11019) as the microorganism in the agar slant medium of Nutrient Broth (NB) in a test tube of 18 mm diameter at 35°C for 24 hours, 1 ml of the suspension in which the cultured bacterium was suspended in 10 ml of sterile water was inoculated to 100 ml of the medium shown in Table 3 placed in a 500 ml-volume flask, and subjected to shake culture at 35°C and 150 rpm for 12 hours. Furthermore, 20 ml of the shake culture solution was divided and inoculated into 2 1 of the medium shown in Table 3 placed in a 5 1-volume fermentation tank to thereby carry out the cultivation for 30 hours under conditions at 35°C, 1000 rpm and airflow rate of 1 1/min. (0.5 vvm), while controlling the pH not less than 7 using 15% ammonia water and controlling the glucose concentration within the range from 5 g/l to 50 g/l by intermittently adding 50% glucose fluid.

**Table 3**

| Components | Final concentration (g/l) | Sterilization conditions |
|---|---|---|
| Peptone | 10.0 | 121°C x 20 minutes |
| Yeast extract | 5.0 | |
| K₂HPO₄ | 5.0 | |
| MgSO₄·7H₂O | 0.5 | |
| Glucose | 20.0 | 121°C x 20 minutes (sterilized separately) |

As a result, 3 1 of the culture solution having the number of cells of 1 x 10¹¹ and turbidity of 105 was obtained. The turbidity was determined by measuring the absorbance at a wavelength of 660 nm using a spectrophotometer (produced by Hitachi, Ltd.; Model U-1800 Ratio Beam Spectrophotometer).

### Example 2: Soil remediation treatment by allowing the soil with the added mixture liquid to stand (35°C)

30 ml each of the liquid additives shown in the entries of Conditions 1 to 4 in Table 4 was added to each of soil samples and mixed at Day 0 and Day 20 in the treatment of allowing 1 kg of soil having total contained CN of 40.0 mg/kg, total eluting CN of 2.2 mg/l and total difficult-to-extract CN of 20 mg/kg (specific gravity of 1.7 kg/l; water content of 25 mass%) placed in a 1 1-volume polyethylene container with a lid to stand in a constant-temperature bath at 35°C for 40 days, and the remediation was observed by measuring total eluting CN, total contained CN and total difficult-to-extract CN of the soil samples.

Results are shown below according to the ranking by soil remediation performance after 40 days.

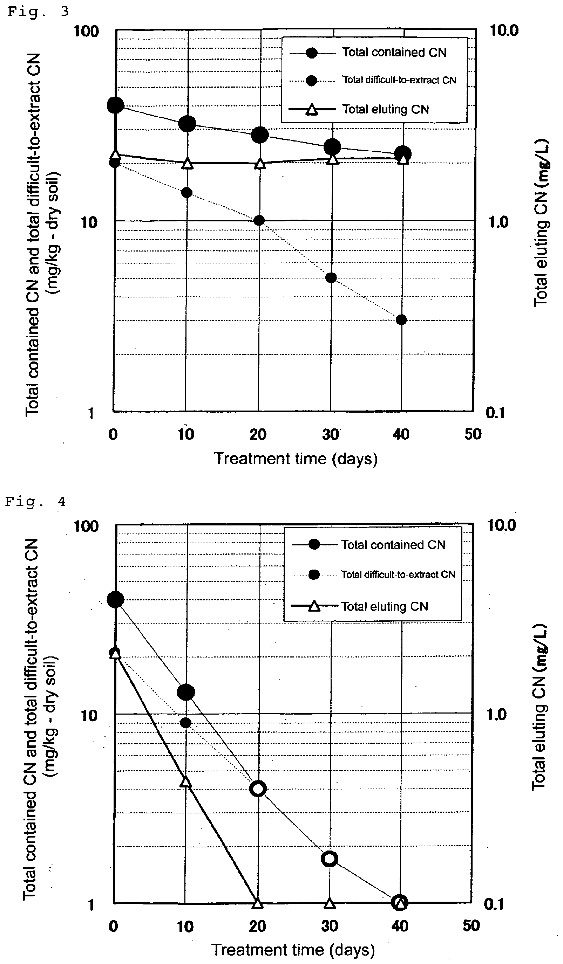

The most favorable condition was Condition 4 in which all of the cyanogen-degrading bacterium, nutrient source and elution promoter were added. The sample was found to have total contained CN of 0.9 mg/kg, total eluting CN < 0.1 mg/l, and total difficult-to-extract CN of 0.9 mg/kg, which passed the standards provided by Soil Contamination Countermeasures Act, and reached a cleanup level such that the risk of the total eluting CN being detected due to the elution from the total contained CN was eliminated. When analyzing the purification tendency, the degradation by the added bacterium and the elution effects by the elution promoter appeared prominently as in Fig. 4, and it was confirmed that the total difficult-to-extract CN was constantly reduced.

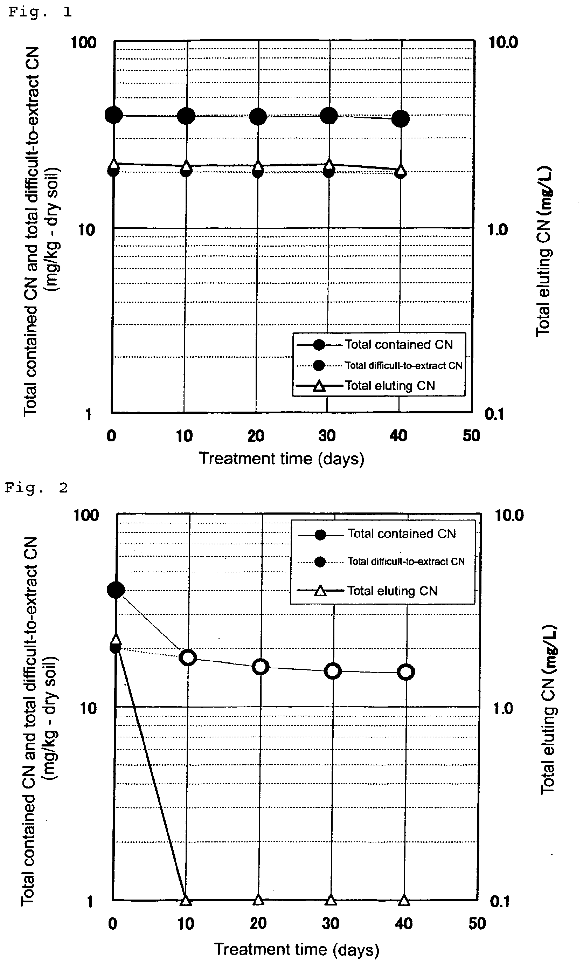

The second-best condition was Condition 2 in which the cyanogen-degrading bacterium and the nutrient source were added. The sample was found to have total contained CN of 15.0 mg/kg, total eluting CN < 0.1 mg/l, and total difficult-to-extract CN of 15.0 mg/kg, which passed the standards provided by Soil Contamination Countermeasures Act, but did not reach a cleanup level such that the risk of the total eluting CN being detected due to the elution from the total contained CN was eliminated. When analyzing the purification tendency, the remediation at the initial stage proceeds at a fast pace owing to the degradation effect by the added bacterium as in Fig. 2. However, after the tenth day, the total contained CN became equal to the total difficult-to-extract CN, and the absence of an elution promoter had an influence. As a result, while the total eluting CN was not detected, the total contained CN did not show a decline, either.

The third-best condition was Condition 3 in which the nutrient source and the elution promoter were added. The sample was found to have total contained CN of 22.0 mg/kg, total eluting CN of 2.1 mg/l, and total difficult-to-extract CN of 3.0 mg/kg. Not only the risk of the total eluting CN being detected due to the elution from the total contained CN remained but the total eluting CN was detected and the soil sample did not pass the standards provided by Soil Contamination Countermeasures Act either. When analyzing the purification tendency, since the cyanogen-degrading bacterium is not added in Condition 3, the degradation by the indigenous bacterium in the soil activated by the added nutrient source continues at a slow pace as in Fig. 3. This is because the difficult-to-extract CN is constantly changed to the soluble CN due to the addition of the elution promoter. However, since the degrading rate is lower than the elution rate, the total eluting CN showed a slight increase.

The worst condition was Condition 1 in which only water was added. The sample was found to have total contained CN of 38.0 mg/kg, total eluting CN of 2.1 mg/l, and total difficult-to-extract CN of 19.6 mg/kg and the soil was found hardly cleaned up from the initial state (see Fig. 1).

### Example 3: Soil mediation treatment by allowing the soil with the added mixture liquid to stand (15°C)

30 ml of the liquid additive shown in the entry of Condition 4 in Table 4 was added to the soil sample and mixed at Day 0 and Day 20 in the treatment of allowing 1 kg of soil having total contained CN of 10.0 mg/kg, total eluting CN of 0.2 mg/l and total difficult-to-extract CN of 8.1 mg/kg (specific gravity of 1.7 kg/l; water content of 25 mass%) placed in a 1 1-volume polyethylene container with a lid to stand in a constant-temperature bath at 15°C for 40 days, and the remediation was observed by measuring total eluting CN, total contained CN and total difficult-to-extract CN of the soil sample.

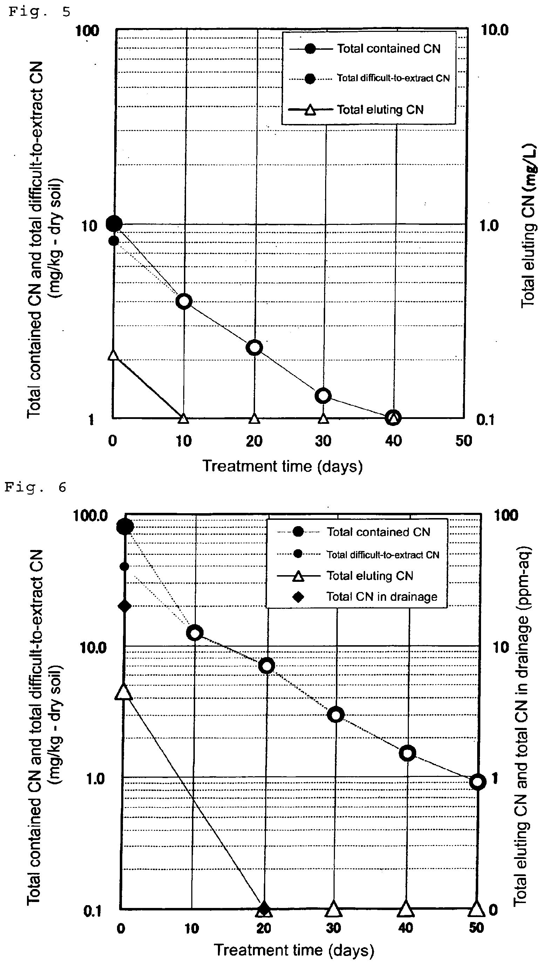

As a result, the sample after 40 days was found to have total contained CN of 0.9 mg/kg, total eluting CN < 0.1 mg/l and total difficult-to-extract CN of 0.9 mg/kg, which passed the standards provided by Soil Contamination Countermeasures Act, and reached a cleanup level such that the risk of the total eluting CN being detected due to the elution from the total contained CN was eliminated (see Fig. 5).

### Example 4: Soil remediation treatment by passing liquid in batches and allowing the soil to stand (at an ambient temperature)

In the treatment of allowing 1 ton (T) of soil having total contained CN of 80.0 mg/kg, total eluting CN of 0.2 mg/l and total difficult-to-extract CN of 39.5 mg/kg (specific gravity of 4.5 kg/l; water content of 30 mass%) placed in a 1 ton-volume container to stand, the operation of pouring 300 1 of the liquid additive shown in the entry of Condition 5 in Table 4 to the soil from the top surface of the container and making 300 1 of the liquid exhausted and recovered from a bottom valve was carried out in two batches at Day 0 and Day 20; and the remediation was observed by measuring total eluting CN, total contained CN and total difficult-to-extract CN of the soil sample. As a result, the soil sample after 50 days of the treatment was found to have total contained CN of 0.9 mg/kg, total eluting CN < 0.1 mg/l, and total difficult-to-extract CN of 0.9 mg/kg, which passed the standards provided by Soil Contamination Countermeasures Act, and reached a cleanup level such that the risk of the total eluting CN being detected due to the elution from the total contained CN was eliminated. Also, CN of the drainage recovered from the bottom valve at passing the liquid was 20 mg/l at the first batch of Day 0, and fell below the detection limit (< 0.1 mg/l) at the second batch of Day 20.

The experiment was carried out at an ambient temperature, and the average temperature was 28°C from Day 0 to Day 20 and 25°C from Day 20 to Day 50.

**[Table 4]**

| Kinds | Components | Conditions | | | | |
|---|---|---|---|---|---|---|
| | | 1 | 2 | 3 | 4 | 5 |
| Degrading bacterium | Arthrobacter sp. No. 5 | 0 cell/ml | 9 x 109 cells/ml | 0 cell/ml | 9 x 109 cells/ml | 2 x 10⁸ cells/ml |
| Nutrient Source | Peptone | 0 g/l | 15 g/l | 15 g/l | 15 g/l | 0.4 g/l |
| | Glucose | 0 g/l | 80 g/l | 80 g/l | 80 g/l | 2.3 g/l |
| Elution promoter | Na₃(C₃H₅O(COO)₃) ·2H₂O | 0 g/l | 0 g/l | 80 g/l | 80 g/l | 2.3 g/l |

### BRIEF DESCRIPTION OF DRAWINGS

[Fig. 1] Fig. 1 shows the changes in the total cyanogen concentration under Condition 1 in Example 2.
[Fig. 2] Fig. 2 shows the changes in the total cyanogen concentration under Condition 2 in Example 2.
[Fig. 3] Fig. 3 shows the changes in the total cyanogen concentration under Condition 3 in Example 2.
[Fig. 4] Fig. 4 shows the changes in the total cyanogen concentration under Condition 4 in Example 2.
[Fig. 5] Fig. 5 shows the changes in the total cyanogen concentration under Condition 4 in Example 3.
[Fig. 6] Fig. 6 shows the changes in the total cyanogen concentration under Condition 5 in Example 4.

## Claims

1. A method for remediation of a soil containing a difficult-to-extract cyanogen compound comprising adding a biodegradable chelating agent to the soil to thereby make the cyanogen compound elute and degrading the eluted cyanogen compound using microorganisms, **characterized in that** the microorganisms are Arthrobacter sp. No. 5 strain having the accession number FERM BP-11019.

2. The method for remediation of a soil as claimed in claim 1, wherein the biodegradable chelating agent to be added is one or more of the members among citric acid, gluconic acid, tartaric acid, oxalic acid, succinic acid, carboxymethyl tartronic acid, carboxymethyloxy succinic acid, aspartate diacetic acid, L-glutamic acid diacetic acid and salts thereof.

3. The method for remediation of a soil as claimed in claim 2, wherein the biodegradable chelating agent to be added is at least one selected from the group consisting of citric acid and citrate.

## Patentansprüche

1. Verfahren zum Verbessern von eine schwer zu extrahierende Cyanogenverbindung enthaltender Erde, welches das Zugeben eines bioabbaubaren Chelatisierungsmittels zu der Erde zur Elution der Cyanogenverbindung und das Abbauen der eluierten Cyanogenverbindung unter Einsatz von Mikroorganismen umfasst, **dadurch gekennzeichnet, dass** die Mikroorganismen der Stamm Arthrobacter sp. Nr. 5 mit der Hinterlegungsnummer FERM BP-11019 sind.

2. Verfahren zum Verbessern von Erde nach Anspruch 1, wobei das zuzugebende bioabbaubare Chelatisierungsmittel ein oder mehrere Mitglieder der Gruppe ist, die aus Zitronensäure, Gluconsäure, Weinsäure, Oxalsäure, Bernsteinsäure, Carboxymethyltartronsäure, Carboxymethyloxybernsteinsäure, Aspartatdiesssigsäure, L-Glutaminsäurediessigsäure und Salzen davon besteht.

3. Verfahren zum Verbessern von Erde nach Anspruch 2, wobei das zuzugebende bioabbaubare Chelatisierungsmittel mindestens eines ist, das aus der aus Zitronensäure und Citrat bestehenden Gruppe ausgewählt ist.

## Revendications

1. Procédé de réhabilitation d'un sol contenant un composé de cyanogène difficile à extraire, comprenant l'addition d'un agent chélatant biodégradable au sol dans le but d'éluer ainsi le composé de cyanogène, et la dégradation du composé de cyanogène élué en utilisant des microorganismes, **caractérisé en ce que** les microorganismes sont la souche Arthrobacter espèce n° 5 dont le numéro d'ordre est FERM ABP-11019.

2. Procédé de réhabilitation d'un sol selon la revendication 1, dans lequel l'agent chélatant biodégradable à ajouter est un ou plusieurs des éléments parmi l'acide citrique, l'acide gluconique, l'acide tartrique, l'acide oxalique, l'acide succinique, l'acide tartronique carboxyméthyle, l'acide succinique carboxyméthyloxy, l'acide diacétique aspartate, l'acide L-glutamique, l'acide diacétique ainsi que des sels de ceux-ci.

3. Procédé de réhabilitation d'un sol selon la revendication 2, dans lequel l'agent chélatant biodégradable à ajouter est au moins un élément parmi le groupe constitué de l'acide citrique et du citrate.
